Europäisches Patentamt

European Patent Office

Office européen des brevets

(11) Veröffentlichungsnummer: **0 075 831**
**A1**

## (12) EUROPÄISCHE PATENTANMELDUNG

(21) Anmeldenummer: 82108685.7

(22) Anmeldetag: 20.09.82

(51) Int. Cl.³: **H 05 G 1/02**
**G 21 K 1/02, A 61 B 6/06**

(30) Priorität: 30.09.81 DE 3138939

(43) Veröffentlichungstag der Anmeldung:
06.04.83 Patentblatt 83/14

(84) Benannte Vertragsstaaten:
BE DE FR

(71) Anmelder: SIEMENS AKTIENGESELLSCHAFT
Berlin und München Wittelsbacherplatz 2
D-8000 München 2(DE)

(72) Erfinder: Wons, Heinz
Humboldtstrasse 10
D-8520 Erlangen(DE)

(54) Röntgenuntersuchungsgerät.

(57) Die Erfindung bezieht sich auf ein Röntgenuntersuchungsgerät mit einer Röntgenröhre (6), einer fokusnahen Primärstrahlenblende (8) und einer in Strahlenrichtung hinter der Patientenlagerungsplatte (21) angeordneten Röntgenaufnahmeeinrichtung (22), mit einem Bildschichtträger (25), einer dem Bildschichtträger in Strahlenrichtung vorgelagerten filmnahen Einblendvorrichtung (29, 56) sowie mit einem Streustrahlenraster (31, 59). Um mit einem solchen Röntgenuntersuchungsgerät auch Röntgenaufnahmen nach dem Schlitzblendenverfahren vornehmen zu können, sieht die Erfindung vor, daß die fokusnahe Primärstrahlenblende (8) Mittel (11, 12, 14, 15, 17,) zur definierten schlitzförmigen Einblendung beinhaltet und die filmnahe Einblendvorrichtung (29, 56) zwei gegeneinander bis hin zu einem minimalen Schlitzabstand verstellbare Blendenplatten (37, 38, 63, 64) beinhaltet und der fokusnahen Primärstrahlenblende sowie der filmnahen Einblendvorrichtung je ein Stelltrieb (18, 47, 39, 40, 45, 65, 66, 67, 68, 69) zur synchronen zueinander ausgerichteten Verstellung zugeordnet sind. Zusätzlich kann den Blendenplatten der filmnahen Einblendvorrichtung (29, 59) mindestens ein Anschlag (48, 49, 70, 71) zugeordnet sein, der eine gegenseitige Annäherung nur bis zu einer vorgegebenen Schlitzbreite zuläßt. Ein erfindungsgemäßes Röntgenuntersuchungsgerät ist insbesondere für den Einsatz in Röntgenabteilungen von Krankenhäusern oder bei Röntgenologen geeignet.

FIG 1

EP 0 075 831 A1

SIEMENS AKTIENGESELLSCHAFT    Unser Zeichen

Berlin und München    VPA 81 P 5092 E

Röntgenuntersuchungsgerät

Die Erfindung bezieht sich auf ein Röntgenuntersuchungsgerät mit einer Röntgenröhre, einer fokusnahen Primärstrahlenblende, einer Patientenlagerungsplatte und einer in Strahlenrichtung hinter der Patientenlagerungsplatte angeordneten Röntgenaufnahmeeinrichtung mit einem Bildschichtträger, mit einer dem Bildschichtträger in Strahlenrichtung vorgelagerten filmnahen Einblendvorrichtung sowie mit einem Streustrahlenraster.

Es ist allgemein bekannt, daß der Kontrastreichtum und das Auflösungsvermögen, die beide die Detailerkennbarkeit von Röntgenaufnahmen bestimmen, durch Verringerung des Streustrahlenanteils gesteigert werden können. Zur Verringerung des Streustrahlenanteils ist es allgemein üblich in Strahlenrichtung unmittelbar vor der Bildschichtebene ein Streustrahlenraster vorzusehen. Alternativ hierzu ist es aber auch bekannt, den Strahlenkegel schlitzförmig einzublenden und mit ihm über das Untersuchungsobjekt und die darunterliegende Röntgenfilmkassette hinwegzustreichen und anstelle des Streustrahlenrasters eine weitere, den selben schmalen Strahlenfächer einblendende Schlitzblende synchron und exakt zum Strahlenfächer ausgerichtet über die Röntgenfilmkassette hinweg zu bewegen. Bei diesem sog. "Schlitzblendenverfahren" werden je Zeiteinheit nicht nur weniger Streustrahlen im Volumen des Untersuchungsobjektes erzeugt, was eine direkte Folge der Verringerung des durchstrahlten Volumens ist. Vielmehr gelangt darüber hinaus von diesen Streustrahlen wegen der synchron über der Röntgenfilmkassette mitgeführten

Stk 5 R1 / 28.09.1981

weiteren Schlitzblende nur ein vernachlässigbarer kleiner Anteil auf das Filmblatt. Dafür müssen der Aufwand für die synchrone Steuerung der beiden Schlitzblenden sowie längere Aufnahmezeiten und eine größere Röhrenbelastung in Kauf genommen werden.

Ein für die Anfertigung von Röntgenaufnahmen nach dem Schlitzblendenverfahren ausgebildetes Röntgenuntersuchungsgerät ist bereits durch das deutsche Gebrauchsmuster 77 10 947 bekannt. Bei diesem Röntgenuntersuchungsgerät läßt sich der Strahlenkegel über eine spezielle Einblendvorrichtung mit zwei Schlitzblenden fächerförmig einblenden und senkrecht zur Fächerebene über den Untersuchungsbereich hinwegführen. In Strahlenrichtung unmittelbar vor der Filmebene ist bei diesem Röntgenuntersuchungsgerät eine weitere Schlitzblende angeordnet, die synchron mit dem fächerförmig eingeblendeten Strahlenkegel und zu diesem ausgerichtet über die Filmebene hinweg verfahrbar ist. Hierzu werden der Motor, der die Einblendvorrichtung verstellt, und der andere Motor, der die filmnahe Schlitzblende verschiebt so gesteuert, daß die Einblendvorrichtung und die filmnahe Schlitzblende stets den gleichen fächerförmigen Strahlenkegel einblenden. Es ist eine Eigenart dieser Konstruktion, daß der Aufwand für die Synchronbewegung der beiden Schlitzblenden mit schmaler werdender Schlitzbreite, d.h. mit den Anforderungen an die Regelgenauigkeit erheblich wachsen. Andererseits verbessert sich die Detailerkennbarkeit gerade um so mehr je schmaler die schlitzförmige Einblendung und damit der Strahlenfächer gehalten werden kann. Schlichßlich bringt es diese Konstruktion mit sich, daß der Arzt der Aufnahmen nach dem Schlitzblendenverfahren erstellen will, sich den notwendigen Raum und auch den finanziellen Aufwand für ein solches Spezielgerät leisten muß.

- 3 -    VPA 81 P 5092  E

Der Erfindung liegt die Aufgabe zugrunde, den mit der Einführung des Schlitzblendenverfahrens für den Arzt verbundenen Aufwand zu verringern.

Bei einem Röntgenuntersuchungsgerät der eingangs genannten Art beinhaltet daher erfindungsgemäß die fokusnahe Primärstrahlenblende Mittel zur definierten schlitzförmigen Einblendung und beinhaltet die filmnahe Einblendvorrichtung zwei gegeneinander bis hin zu einem minimalen, der schlitzförmigen Einblendung der fokusnahen Primärstrahlenblende angepaßten Schlitzabstand verstellbare Blendenplatten und ist der fokusnahen Primärstrahlenblende sowie der filmnahen Einblendvorrichtung je ein Stelltrieb zur synchronen, zueinander ausgerichteten Verstellung der fokusnahen und filmnahen schlitzförmigen Einblendmittel quer zur Schlitzrichtung zugeordnet..Dies hat zur Folge, daß mit ein und demselben Röntgenuntersucnungsgerät sowohl konventionelle Röntgenaufnahmen als auch Aufnahmen nach dem Schlitzblendenverfahren erstellbar sind.'Für den Kunden entfällt so der Ankauf eines separaten Röntgenuntersuchungsgerätes und die Notwendigkeit entsprechenden Raum für die Aufstellung dieses zweiten Röntgenuntersuchungsgerätes bereitzustellen.

In zweckmäßiger Weiterbildung der Erfindung kann den Blendenplatten der filmnahen Einblendvorrichtung mindestens ein Anschlag zugeordnet sein, der eine gegenseitige Annäherung nur bis zu einer vorgegebenen Schlitzbreite zuläßt. Hierdurch wird eine exakte Einstellung des filmnahen Schlitzes in einfacher Weise sichergestellt. Damit ist eine Voraussetzung für die Minimalisierung des Streustrahlenanteils erfüllt.

Die Einstelltechnik läßt sich weiter vereinfachen, wenn in zweckmäßiger Ausgestaltung der Erfindung den Blendenplatten der filmnahen Einblendvorrichtung mindestens eine sie aufeinander zu ziehende Feder zugeordnet ist. Hierdurch wird es möglich, eine exakte Einstellung der Blendenplatten auf Schlitzbreite durch bloßes Entkoppeln der Stelltriebe zu erreichen.

In vorteilhafter Ausgestaltung der Erfindung kann den Blendenplatten der filmnahen Einblendvorrichtung ein Stelltrieb zugeordnet sein, über den sie aufeinander zu oder voneinander weg bewegbar sind. So wird ohne nennenswerten Aufwand auch die Einblendung konventioneller Filmformate ermöglicht.

In besonders vorteilhafter Weiterbildung der Erfindung kann den Blendenplatten der filmnahen Einblendvorrichtung ein Stelltrieb zugeordnet sein, über den sie bei unverändertem gegenseitigen Abstand gemeinsam senkrecht zu ihren den Strahlenkegel begrenzenden Kanten verstellbar sind. Hiermit wird eine Voraussetzung geschaffen, um so mit den gleichen Stellmitteln sowohl Aufnahmen nach dem Schlitzblendenverfahren als auch unterteilte konventionelle Aufnahmen zu erstellen.

Eine erhebliche Konstruktionsvereinfachung läßt sich erreichen, wenn in besonders zweckmäßiger Ausgestaltung der Erfindung eine lösbare Kupplung in den der einen filmnahen Blendenplatte zugeordneten Antriebsweg eingebaut ist. Diese Kupplung ermöglicht es, im Zusammenhang mit der die Blendenplatten aufeinander zu ziehende Feder, durch bloßes Betätigen vom konventionellen Aufnahmebetrieb zum Schlitzblendentrieb zu wechseln.

Weitere Einzelheiten der Erfindung werden anhand der in den Figuren dargestellten Ausführungsbeispiele erläutert. Es zeigen:

Fig. 1 eine schematische Darstellung eines erfindungsgemäßen Untersuchungsgerätes in Seitenansicht,

Fig. 2 eine schematische Darstellung der Stelltriebe für die Blendenplatten des in der Fig. 1 dargestellten Röntgenzielgerätes,

Fig. 3 eine schematische Darstellung der Position der Blendenplatten im Röntgenzielgerät der Fig. 1 bei der Erstellung von unterteilten Röntgenaufnahmen nach dem Schlitzblendenverfahren,

Fig. 4 eine Darstellung der Anordnung der einzelnen Meßkammern in der in der Fig. 1 dargestellten Ionisationskammer und

Fig. 5 eine schematische Darstellung der Stelltriebe für die Blendenplatten bei einem abgewandelten erfindungsgemäßen Röntgenzielgerät.

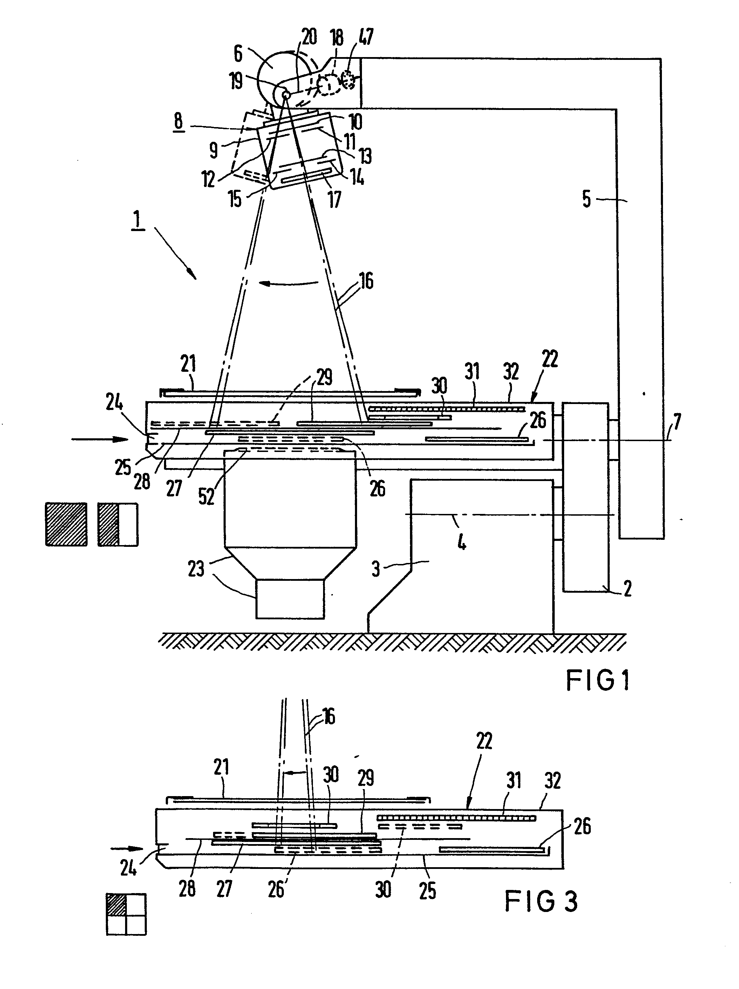

Die schematische Darstellung der Fig. 1 zeigt ein Röntgenuntersuchungsgerät 1, dessen Tischgestell 2 am Fußgestell 3 um eine horizontale Kippachse 4 schenkbar gelagert ist. Am Tischgestell 2 ist eine Tragsäule 5 für eine Röntgenröhre 6 um eine weitere horizontale Achse 7, die parallel zur Kippachse 4 ausgerichtet ist, schwenkbar gelagert. An der Röntgenröhre ist eine Primärstrahlenblende 8 angeflanscht. Im Gehäuse 9 der

Primärstrahlenblende 8 sind die Blendenplatten 10, 11, 12, 13, 13, 15 mit denen das Strahlenfeld 16 eingeblendet werden kann, sowie eine weitere aus dem Strahlenfeld herausziehbare fokusnahe Schlitzblende 17 untergebracht. Der Röntgenröhre 6 ist ein Stellmotor 18 zugeordnet, über den sie mitsamt der Primärstrahlenblende 8 um eine durch ihren Fokus 19 gehende Achse 20 geschwenkt werden kann. Auf dem Tischgestell ist eine Patientenlagerungsplatte 21 in hier nicht weiter dargestellter Weise verschiebbar gelagert. Im Tischgestell 2 ist ein Röntgenzielgerät 22 eingebaut. Das Röntgenzielgerät trägt auf seiner Unterseite eine Bildverstärker-Fernseheinrichtung 23. Auf der in der Darstellung der Fig. 1 linken Seite des Röntgenzielgerätes 22 erkennt man eine Kasetteneinschuböffnung 24 und eine Transportbahn 25 für eine Röntgenfilmkassette 26, die in der Bereitschaftsposition auf der Transportbahn liegend, eingezeichnet ist. Oberhalb der Transportbahn 25 für die Röntgenfilmkassette 26 befindet sich in der Aufnahmeposition, d.h. im Bereich des einblendbaren Strahlenkegels 16 eine Ionisationskammer 27. Oberhalb der Ionisationskammer ist in einer weiteren Transportbahn 28 eine parallel zur Kassettenebene verschiebbare filmnahe Schlitzblende 29 zu erkennen. In Strahlenrichtung vor der filmnahen Schlitzblende befindet sich eine bei Bedarf aus der Bereitschaftsposition in die Aufnahmeposition verschiebbare rahmenartige Blendenplatte 30. In Strahlenrichtung oberhalb der rahmenartigen Blendenplatte ist ein in der Bereitschaftsposition geparktes Streustrahlenraster 31 zu erkennen.

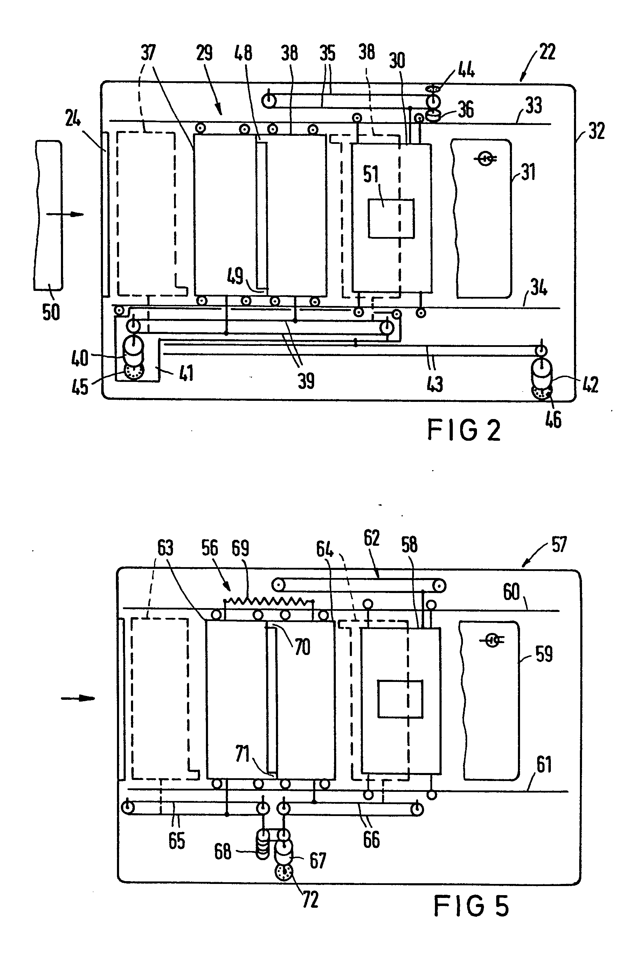

Die Fig. 2 zeigt in einer schematischen Darstellung eine Aufsicht auf den Stellmechanismus für die filmnahe Schlitzblende 29 und die rahmenartige Blendenplatte 30. Die filmnahe Schlitzblende und die rahmenartige Blendenplatte sind im Zielgerätegehäuse 32 längs zweier paralleler Schienen 33, 34 von einer Bereitschaftsposition in eine Aufnahmeposition und wieder zurück verfahrbar. Dazu ist die rahmenartige Blendenplatte an einen endlosen Zahnriemen 35 angehängt, der von einem Motor 36 angetrieben ist. Die beiden Blendenplatten 37, 38 der filmnahen Schlitzblende 29 sind eine jede für sich an je einen Trumm eines weiteren endlosen Zahnriemens 39 angekuppelt. Dieser Zahnriemen ist mitsamt den ihn antriebenden Motor 40 auf einer separaten Platine 41 gelagert. Diese Platine ist ihrerseits längs der unteren Schiene 34 im Zielgerätegehäuse 32 verfahrbar. Zu diesem Zweck ist sie an einen dritten von einem Motor 42 angetriebenen endolsen Zahnriemen 43 angehängt. Alle drei Motoren 36, 40, 42 wie auch der Stellmotor 18 für die Schwenkung der Röntgenröhre 6 tragen auf ihrer Achse je eine Lochscheibe 44, 45, 46, 47,deren Löcher zwischen Lichtquelle und Fotodetektor je einer Lichtschranke (der übersichtlichkeithalber nicht dargestellt) hindurchgedreht werden können.

Die Blendenplatten 37, 38 der filmnahen Schlitzblende 29 tragen an ihren einander zugewandten Kanten im jeweiligen Randbereich je einen fortsatzartigen Anschlag 48, 49 durch den ihr gegenseitiger minimaler, die Schlitzbreite bestimmender Abstand festgelegt ist. Vor der Kasetteneinschuböffnung 24 ist zur Verdeutlichung eine einzuschiebende Röntgenfilmkassette 50 zu erkennen. In der Bereitschaftsposition ist das Streustrahlenraster 31 angedeutet.

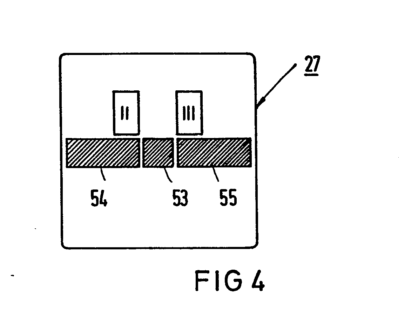

Die Fig. 3 zeigt das Röntgenzielgerät 22 der Fig. 1 in einer Betriebsphase, bei der eine Vierfachunterteilung des in der Röntgenfilmkassette 26 eingesetzten Filmblattes vorgenommen wird. Die Vierfachunterteilung ist neben der Fig. 3 schematisch  angedeutet. In diesem Fall ist die rahmenartige Blendenplatte 30 zusätzlich zu den Blendenplatten 37, 38 der filmnahen Schlitzblende 29 in die Aufnahmeposition eingefahren. Die rahmenartige Blendenplatte verhindert, daß die Bereiche der Röntgenfilmkassette 26, die außerhalb des Durchbruchs 51 der rahmenartigen Blendenplatte liegen, belichtet werden können. Bei vierfacher Unterteilung des Filmformates muß dann dieses Teilfeld des Filmblattes bzw. der Röntgenfilmkassette zum Zentrum des Durchbruchs 51 der rahmenartigen Blendenplatte zentriert werden. Dies erfordert, bezogen auf die Fig. 3, u.a. auch eine Verschiebung der Röntgenfilmkassette 26 senkrecht zur Zeichenebene. Die Röntgenstrahlung wird in diesem Fall erst eingeschaltet, nachdem ein Schwenkwinkel der fokusnahen Primärstrahlenblende 8 und eine Position der auf Schlitzabstand angenäherten filmnahen Blendenplatten 37, 38 erreicht ist, bei dem er fächerförmige Strahlenkegel 16 den Durchbruch 51 der rahmenartigen Blendenplatte 30 zu überstreichen beginnt. Die Röntgenstrahlung wird abgeschaltet, sobald der fächerförmige Strahlenkegel die gegenüberliegende Seite des Durchbruchs in der rahmenartigen Blendenplatte erreicht hat.

Die Fig. 4 zeigt eine Aufsicht auf die Ionisationskammer 27 über die der Röhrenstrom geregelt wird. Diese Ionisationskammer entspricht einer vorbekannten DreiFelder-Ionisationskammer, jedoch sind zusätzlich beidseitig des zentralen Meßfeldes 53 noch zwei schmale Meßfelder 54, 55 ergänzt worden. Bei Schlitzblendenbetrieb werden diese beiden zusätzlichen Meßfelder und das zentrale Meßfeld 53 eingeschaltet. Infolge der gleichmäßigen Breite der Felder kann so die Dosisleistung während des gesamten Bewegungsablaufs der
fokusnahen Primärstrahlenblende 8 und der filmnahen
Schlitzblende 29 entsprechend der Objektdicke  nachgeregelt werden.

Soll mit dem in den Fig. 1 bis 3 dargestellten Röntgenuntersuchungsgerät 1 eine konventionelle Röntgenaufnahme erstellt werden, so wird eine Röntgenfilmkassette 26 durch die Kassetteneinschuböffnung 24
eingeschoben und in hier nicht weiter dargestellter
Weise längs der Transportbahn 25 in die Bereitschaftsposition unterhalb des in der Fig. 1 in Bereitschaftsstellung eingezeichneten Streustrahlenrasters 31 verfahren und dort geparkt. Die Breite des Aufnahmefeldes
kann durch entsprechendes Verstellen der Blendenplatten
10, 11, 12, 13, 14, 15 der Primärstrahlenblende 8 bei
gleichzeitig aus dem Strahlenfeld herausgezogener
fokusnaher Schlitzblende 17 und durch synchrone gegenläufige Verstellung der beiden Blendenplatten 37, 38
der filmnehan Schlitzblende 29 durch den Motor 40 in
an sich bekannter Weise eingestellt werden. Bei Bedarf
kann durch zusätzliches Einfahren der rahmenartigen
Blendenplatte 30 auch eine zusätzliche Unterteilung
bzw. Eingrenzung des Aufnahmeformats in der Höhe vorgenommen werden. Der Streustrahlenanteil kann in
diesem Fall nur durch das in die Aufnahmeposition eingefahrene Streustrahlenraster 31 heruntergedrückt
werden.

Soll dagegen mit demselben in den Fig. 1 bis 3 dargestellten Röntgenuntersuchungsgerät 1 eine Röntgenaufnahme nach dem Schlitzblendenverfahren erstellt werden,
so kann das Streustrahlenraster 31 in der Bereitschaftsposition verbleiben. Stattdessen wird die Schlitzblende 17 in das Strahlenfeld der fokusnahen Primärstrahlenblende 8 eingeschoben, werden die Blendenplatten 37, 38 der filmnahen Schlitzblende 29 vom Motor 40 in einen durch die Anschläge 48, 49 vorgegebenen geringstmöglichen gegenseitigen Abstand transportiert und wird die Platine 41 mitsamt dem Motor 40 und den Blendenplatten 37, 38 von dem zugehörigen Motor 42 in eine Extremposition gefahren. Auch die fokusnahe Primärstrahlenblende 8 wird vom zugeordneten Stellmotor 17 mitsamt der Röntgenröhre 6 in eine Extremposition gedreht, bei der    von der fokusnahen Schlitzblende 17 ausgeblendete Strahlenfächer durch den Schlitz zwischen den beiden filmnahen Blendenplatten 37, 38 geht. Von dieser Ausgangslage aus wird die Platine 41 mitsamt dem Motor 40 und den auf Anschlag gefahrenen Blendenplatten 37, 38 über die in der Aufnahmestellung befindliche Röntgenfilmkassette hinwegbewegt und wird synchron dazu die fokusnahe Primärstrahlenblende 8 mitsamt der Röntgenröhre 6 so geschwenkt, daß der Strahlenfächer der Primärstrahlenblende stets zum Schlitz zwischen den beiden filmnahen Blendenplatten 37, 38 zentriert ist. Die Synchronisation der beiden Motoren erfolgt in hier nicht weiter dargestellter Weise über die mit diesen Motoren gekuppelten Lochscheiben, die zwischen jeweils einer Lichtschranke drehen. Die in den Fotodetektoren dieser Lichtschranken gezählten Impulse werden in hier nicht weiter dargestellter Weise zur Synchronisation der Motoren 18, 42 herangezogen. Die Leistung der Röntgenröhre kann durch die Ionisationskammer 27 gesteuert werden. Dabei sind lediglich die vom Strahlenfächer überstrahlten beiden schmalen Meßfelder 54, 55 sowie das zentrale Meßfeld 53 eingeschaltet.

Soll bei dieser Röntgenaufnahme das gesamte in der Röntgenfilmkassette 26 eingelegte Filmblatt belichtet werden, so wird dazu die Röntgenfilmkassette, wie in der Fig. 1 punktiert dargestellt, zentrisch über den Eingangsleuchtschrim 52 des Röntgenbildverstärkers positioniert. Soll jedoch eine Zweifachunterteilung des Filmformates vorgenommen werden, so darf die Röntgenfilmkassette, so wie das in der Fig. 3 punktiert dargestellt ist, nur soweit in die Aufnahmeposition eingefahren werden, daß das zu belichtetnde Teilfeld zentrisch zum Aufnahmebereich bzw. zentrisch zur Mittelsenkrechten auf den stets zum Aufnahmebereich zentrierten Röntgenbildverstärker steht. Die Röntgenröhre wird in diesem Fall nur in dem Schwenkbereich eingeschaltet, in dem der ausgeblendete Strahlenfächer das zu belichtende Feld der Röntgenfilmkassette überstreicht, d.h. die Verschiebung der filmnahen Schlitzblende und der synchron mit verschobenen fokusnahen Primärstrahlenblnde erfolgt nur in diesem Bereich.

Die Fig. 5 zeigt eine Variante des in der Fig. 2 abgebildeten Stelltriebs für die filmnahe Schlitzblende 56. Während bei diesem Röntgenzielgerät 57 die filmnahe Schlitzblende 56, die rahmenartige Blendenplatte 58, die Streustrahlenblende 59 sowie die zugehörigen Schienen 60, 61 und der Stelltrieb 62 für die rahmenartige Blendenplatte 58 unverändert geblieben sind, sind die Platine mit dem Motor und dem Zahnriemen entfallen. Stattdessen sind für die gegenläufige Verstellung der beiden Blendenplatten 63, 64 der filmnahen Schlitzblende 56 zwei separate endlose Zahnriemen 65, 66 vorgesehen, die von ein und demselben Motor 67 synchron und gleichsinnig angetrieben werden. In dem Übertragungsweg der einen Blendenplatte 63 ist dafür eine abschaltbare Kupplung 68 eingebaut. Außerdem werden die beiden Blendenplatten 63, 64 der filmnahen Schlitzblende 56 durch eine Zugfeder 69 aufeinander zu gezogen.

Mit dem Motor 67 können bei eingeschalteter Kupplung 68 die beiden Blendenplatten 63, 64 gegensinnig verstellt werden. Dadurch kann das Aufnahmefeld bei konventionellen Röntgenaufnahmen nach Belieben in seiner Breite variiert werden. Beim Übergang zur Schlitzaufnahmetechnik braucht hier lediglich die Kupplung 68 nach der motorischen Annäherung der Blendenplatten gelöst zu werden. Dadurch werden die beiden Blendenplatten 63, 64 von der Zugfeder 69 mit ihren Anschlägen 70, 71 in Anlage gehalten. Die beiden so auf Schlitzabstand gebrachten Blendenplatten 63, 64 können so gemeinsam von dem Motor 67 bei gelöster Kupplung 68 quer zur Schlitzrichtung verschoben werden. Diese Konstruktion hat den Vorteil, daß man nur einen einzigen Motor braucht, um entweder bei konventioneller Technik die Breite des Aufnahmefeldes zu variieren oder um beim Schlitzblendenaufnahmebetrieb die aneinanderliegenden Blendenplatten zusammen mit dem fächerförmig eingeblendeten Strahlenkegel über das Aufnahmefeld hinwegzubewegen.

Es wäre auch denkbar, die an der Röntgenröhre angeflanschte Primärstrahlenblende so zu steuern, daß ihre Blendenplatten den fächerförmigen Strahlenkegel ausblenden und dafür auf die separate fokusnahe Schlitzblende zu verzichten. Statt der erwähnten Zahnriementriebe lassen sich auch Kettentriebe verwenden.

5 Figuren

12 Patentansprüche

Patentansprüche

1. Röntgenuntersuchungsgerät mit einer Röntgenröhre, einer fokusnahen Primärstrahlenblende, einer Patientenlagerungsplatte und einer in Strahlenrichtung hinter der Patientenlagerungsplatte angeordneten Röntgenaufnahmeeinrichtung mit einem Bildschichtträger, mit einer dem Bildschichtträger in Strahlenrichtung vorgelagerten filmnahen Einblendvorrichtung sowie mit einem Streustrahlenraster, d a d u r c h  g e k e n n z e i c h - n e t ,   daß die fokusnahe Primärstrahlenblende (8) Mittel (11, 12, 14, 15, 17) zur definierten schlitzförmigen Einblendung beinhaltet und die filmnahe Einblendvorrichtung (29, 56) zwei gegeneinander bis hin zu einem minimalen, der schlitzförmigen Einblendung der fokusnahen Primärstrahlenblende angepaßten Schlitzabstand verstellbare Blendenplatte (37, 38, 63, 64) beinhaltet und der fokusnahen Primärstrahlenblende sowie der filmnahen Einblendvorrichtung je ein Stelltrieb (18, 47, 39, 40, 45, 65, 66, 67, 68, 69) zur synchronen zueinander ausgerichteten Verstellung der fokusnahen und filmnahen schlitzförmigen Einblendmittel quer zur Schlitzrichtung zugeordnet ist.

2. Röntgenuntersuchungsgerät nach Anspruch 1, d a - d u r c h  g e k e n n z e i c h n e t ,   daß den Blendenplatten (37, 38, 63, 64) der filmnahen Einblendvorrichtung (29, 59) mindestens ein Anschlag (48, 49, 70, 71) zugeordnet ist, der eine gegenseitige Annäherung nur bis zu einer vorgegebenen Schlitzbreite zuläßt.

3. Röntgenuntersuchungsgerät nach Anspruch 2, d a - d u r c h  g e k e n n z e i c h n e t ,   daß den Blendenplatten (63, 64) der filmnahen Einblendvorrichtung (56) mindestens eine sie aufeinander zu ziehende Feder (69) zugeordnet ist.

4. Röntgenuntersuchungsgerät nach Anspruch 1,  d a -
d u r c h   g e k e n n z e i c h n e t ,   daß den
Blendenplatten (37, 38, 63, 64) der filmnahen Einblendvorrichtung (29, 56) ein Stelltrieb (39, 40, 45, 46, 65,
66, 67, 68) zugeordnet ist, über den sie aufeinander zu
oder voneinander weg verstellbar sind.

5. Röntgenuntersuchungsgerät nach Anspruch 1,  d a -
d u r c h   g e k e n n z e i c h n e t ,   daß den
Blendenplatten (37, 38, 63, 64) der filmnahen Einblendvorrichtung (29, 56) ein Stelltrieb (42, 43, 46, 66, 67,
69) zugeordnet ist, über den sie bei unverändertem gegenseitigen Abstand gemeinsam senkrecht zu ihren den Strahlenkegel  begrenzenden Kanten verstellbar sind.

6. Röntgenuntersuchungsgerät nach Anspruch 3 und 4,
d a d u r c h   g e k e n n z e i c h n e t ,   daß
eine lösbare Kupplung (68) in dem der einen filmnahen
Blendenplatte (63) zugeordneten Antriebsweg (65, 67)
eingebaut ist.

7. Röntgenuntersuchungsgerät nach Anspruch 1,  d a -
d u r c h   g e k e n n z e i c h n e t ,   daß die
fokusnahe Primärstrahlenblende (8) an der Röntgenröhre
(6) angeflanscht ist und gemeinsam mit dieser über einen
Stellmotor (18) quer zu der durch die schlitzförmige
Einblendvorrichtung (17) gegebene Ebene des Strahlenfächers (16) um einen durch den Fokus (19) der Röntgenröhre gehende Achse (20) schwenkbar gelagert ist.

8. Röntgenuntersuchungsgerät nach Anspruch 1 oder 5,
d a d u r c h   g e k e n n z e i c h n e t ,   daß
die Stellgeschwindigkeit des Stelltriebes (18, 47)
für die fokusnahe Primärstrahlenblende und des Stelltriebs (39, 40, 45, 67, 66, 69) für die gemeinsame Verschiebung der Blendenplatten (37, 38, 63, 64) der filmnahen Einblendvorrichtung (29, 56) bei unveränderten gegenseitigen Abstand in einem fest vorgegebenen Verhältnis zueinander stehen.

9. Röntgenuntersuchungsgerät nach Anspruch 8,  d a - d u r c h   g e k e n n z e i c h n.e t ,  daß die die beiden Stelltriebe antreibenden Motoren (18, 42, 67) je eine Lochscheibe (47, 46, 72) antreiben, deren Löcher zwischen Lichtquelle und Fotodetektor einer Lichtschranke hindurch drehbar sind und die Signale der Fotodetektoren zur Synchronisation der Drehzahlen der Motoren herangezogen sind.

10. Röntgenuntersuchungsgerät nach Anspruch 1,  d a - d u r c h   g e k e n n z e i c h n e t ,  daß mindestens eine weitere, das Strahlenfeld (16) quer zur Schlitzrichtung begrenzende Blendenplatte (30, 58) in Strahlenrichtung vor den filmnahen Blendenplatten (37, 38, 63, 64) einbringbar ist.

11. Röntgenuntersuchungsgerät nach Anspruch 1,  d a - d u r c h   g e k e n n.z.e i c h n e t ,  daß eine zur Ausblendung unterschiedlicher Formate ausgelegte konventionelle fokusnahe Primärstrahlenblende (8) um eine in den Strahlengang einschiebbare, geschlitzte Blendenplatte (17) ergänzt ist.

12. Röntgenuntersuchungsgerät nach Anspruch 1,  d a - d·u r c h   g e k e n n.e t ,  daß die Ionisationskammer (27) für die Belichtungssteuerung beim Schlitzaufnahmebetrieb um zwei schmale zuschaltbare,  quer zur Schlitzreichtung ausgerichtete Meßkammern (54, 55) erweitert ist.

FIG 1

FIG 3

FIG 2

FIG 5

FIG 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | --- | | H 05 G 1/02 |
| A | US-A-4 203 037 (D. GUR et al.) * Spalte 1, Zeilen 9-28; Spalte 2, Zeilen 5-25, 34-62; Spalte 4, Zeile 39 - Spalte 5, Zeile 13; Spalte 8, Zeilen 4-39 * | 1,8 | G 21 K 1/02 A 61 B 6/06 |
| | --- | | |
| A | US-A-4 097 748 (J. MONVOISIN) * Spalte 1, Zeilen 43-65; Spalte 6, Zeile 41 - Spalte 7, Zeile 17 * | 1,8,10 | |
| | --- | | |
| A | DE-A-2 732 073 (TOKYO SHIBAURA ELECTRIC CO., LTD.) * Seite 1, Zeile 1 - Seite 2, Zeile 10 * | 1,4 | |
| | --- | | **RECHERCHIERTE SACHGEBIETE (Int. Cl. ³)** |
| A | DE-A-2 814 242 (SIEMENS AG) * Seite 1, Zeilen 3-30 * | 1 | |
| | --- | | |
| A | ELECTRO MEDICA, Band 49, Nr. 1, Januar 1981, Seiten 34-41, Erlangen, DE. R. MOORE et al.: "Schlitzaufnahmetechnik mit mitgeführtem Strahler" | 1 | A 61 B 6/06 G 21 K 1/02 G 21 K 1/04 H 05 G 1/02 |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 06-01-1983 | HORAK G.I. |